(19) Europäisches Patentamt
European Patent Office
Office européen des brevets

(11) Veröffentlichungsnummer: **0 007 589**
**B1**

(12) # EUROPÄISCHE PATENTSCHRIFT

(45) Veröffentlichungstag der Patentschrift: **13.05.81**

(21) Anmeldenummer: **79102557.0**

(22) Anmeldetag: **20.07.79**

(51) Int. Cl.³: **A 01 N 43/64,**
A 01 N 47/10,
C 07 D 249/08
//(A01N47/10, 43/64)

(54) **Insektizide Mittel, enthaltend Carbamate als Wirkstoffe und Triazolderivate als Synergisten.**

(30) Priorität: **28.07.78 DE 2833193**

(43) Veröffentlichungstag der Anmeldung:
**06.02.80 Patentblatt 80/3**

(45) Bekanntmachung des Hinweises auf die Patenterteilung:
**13.05.81 Patentblatt 81/19**

(84) Benannte Vertragsstaaten:
**AT BE CH DE FR GB IT LU NL SE**

(56) Entgegenhaltungen:
**FR - A - 2 329 660**
**FR - A - 2 367 068**
**FR - A - 2 370 431**

(73) Patentinhaber: **BASF Aktiengesellschaft**
**Carl-Bosch-Strasse 38**
**D-6700 Ludwigshafen (DE)**

(72) Erfinder: **Linhart, Friedrich, Dr. Chem.**
**Leisberg 61**
**D-6900 Heidelberg (DE)**
Erfinder: **Zeeh, Bernd, Dr. Chem.**
**Thorwaldsenstrasse 5**
**D-6700 Ludwigshafen (DE)**
Erfinder: **Adolphi, Heinrich, Dr.**
**Kalmitweg 11**
**D-6703 Limburgerhof (DE)**

**EP 0 007 589 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Insektizide Mittel, enthaltend Carbamate als Wirkstoffe und Triazolderivate als Synergisten

Die Erfindung betrifft insektizide Mittel, die insektizide Wirkstoffe aus der Gruppe der Carbamate und zur Steigerung der insektiziden Wirksamkeit dieser Wirkstoffe Triazolderivate enthalten.

Es ist bekannt, daß beim Einsatz chemischer Wirkstoffe zur Bekämpfung schädlicher Organismen in einigen Fällen bei Mischungen ein wesentlich größerer Effekt erzielt wird als aus der Addition der Einzelwirkungen zu erwarten war. Derartige Wirkungssteigerungen bezeichnet man als Synergismen oder Synergistenwirkungen. Es ist dabei nicht erforderlich, daß der zugesetzte Synergist allein eine erkennbare Wirking auf den Zielorganismus ausübt.

Bei der Bekämpfung schädlicher Insekten verwendet man in der Praxis fast ausschließlich Piperonalderivate als Synergisten. Von Bedeutung ist insbesondere das Piperonylbutoxid, welches u.a. zur Steigerung der Wirking von Pyrethrinen eingesetzt wird.

Außerdem ist aus der FR—PS 2 370 471 bekannt, daß 1-n-Dodecyl-1,2,4-triazol die insektizide Wirkung von Pyrethroiden in synergistischer Weise steigert.

Es wurde nun gefunden, daß insektizide Mittel, die eine insektizid wirksame Menge einer Mischung aus einem Triazolderivate der Formel I

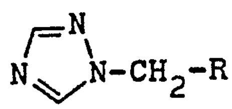

I

in der

R gegebenenfalls durch Halogen substituiertes Phenyl oder den Rest

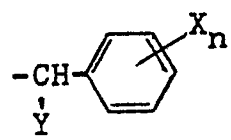

bedeutet,

wobei

Y für Wasserstoff, Halogen, Alkylmercapto mit 1 bis 6 Kohlenstoffatomen oder Alkenylmercapto mit 2 bis 6 Kohlenstoffatomen oder einen gegebenenfalls durch Halogen substituierten Phenylmercaptorest,

X für Halogen und

n für 0, 1 oder 2 stehen, mit der Maßgabe, daß n nicht O bedeutet, wenn Y für Halogen steht, oder einem Salz eines Triazolderivats der Formel I und einem insektiziden Wirkstoff aus der Gruppe der Carbamate enthalten,

eine erheblich größere insektizide Wirksamkeit haben als insektizide Mittel, die die Einzelkomponenten enthalten.

Die Triazolderivate der Formel I und ihre Salze sind sehr stark und breit wirksame Synergisten.

Als synergistisch wirkende Triazolderivate der Formel I kommen 1,2,4-Triazole oder deren Salze in Betracht, die in 1-Stellung einen gegebenenfalls substituierten Benzyl- oder 2-Phenyl-äthylrest tragen, wobei der Phenylrest durch Halogen, insbesondere Chlor oder Fluor, einfach oder mehrfach substituiert sein kann. Ist der Phenylrest mehrfach substituiert, können die Substituenten verschieden sein. Die 2-Phenyläthylreste können in 2-Stellung am Äthylrest darüberhinaus durch Alkylmercaptoreste mit 1 bis 6 Kohlenstoffatomen, insbesondere n-Butylmercapto, Isobutylmercapto, durch Alkenylmercaptoreste mit 2 bis 6 Kohlenstoffatomen, insbesondere Allylmercapto, durch Phenylmercaptoreste, die am Phenylring Halogensubstituenten, insbesondere Chlor, tragen können, oder durch Halogen, insbesondere Chlor, substituiert sein. Ist der 2-Phenyläthylrest in 2-Stellung durch Halogen substituiert, trägt der Phenylring mindestens einen Halogensubstituenten.

Als Salze kommen Salze der Triazolderivate der Formel I mit anorganischen oder organischen Säuren in Betracht. Beispiele für solche Säuren sind Halogenwasserstoffsäuren, insbesondere Chlorwasserstoff, Bromwasserstoff, Salpetersäure, Schwefelsäure, Oxalsäure, Trichloressigsäure, Alkyl- oder Arylsulfonsäuren, insbesondere p-Toluolsulfonsäure, Dodecylbenzolsulfonsäure.

Beispiele für als Synergisten geeignete Triazolderivate sind 1 - [2 - Chlor - 2 - (4 - fluorphenyl) - äthyl] - 1,2,4 - triazol, 1 - [2 - Chlor - 2 - (4 - chlorphenyl) - äthyl] - 1,2,4 - triazol, 1 - [2 - Chlor - 2 - (2,4 - dichlorphenyl) - äthyl] - 1,2,4 - triazol, 1 - [2 - n - Butylmercapto - 2 - (2,4 - dichlorphenyl) - äthyl] - 1,2,4 - triazol, 1 - [2 - Isobutylmercapto - 2 - (2,4 - dichlorphenyl) - äthyl] - 1,2,4 - triazol, 1 - [2 - Allylmercapto - 2 - (2,4 - dichlorphenyl) - äthyl] - 1,2,4 - triazol, 1 - [2 - Brom - 2 - (2,4 - dichlorophenyl) - äthyl] - 1,2,4 - triazol, 1 - Benzyl - 1,2,4 - triazol, 1 - (4 - Chlor-benzyl) - 1,2,4 - triazol, 1 - (2 - Chlor-benzyl) - 1,2,4 - triazol, 1 -

(2 - Phenyl-äthyl) - 1,2,4 - triazol, 1 - [2 - (4 - Chlorbenzyl) - mercapto - 2 - (2,4 - dichlorphenyl) - äthyl] - 1,2,4 - triazol oder Salze, indbesondere Hydrochloride, dieser Triazolderivate.

Triazolderivate der Formel I, in der R den Rest

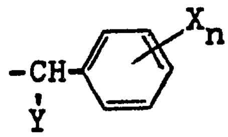

bedeutet, wobei Y und X für Halogen und n für 1 oder 2 stehen, ihre Herstellung und Verwendung als Fungizide sind aus der DE—OS 25 47 954 bekannt.

Triazolderivate der Formel I, in der R für gegebenenfalls durch Halogen substituiertes Phenyl steht, können in an sich bekannter Weise durch Umsetzung von 1,2,4-Triazol mit Benzylhalogeniden, die am Phenylring gegebenenfalls durch Halogen substituiert sind, erhalten werden.

Triazolderivate der Formel I, in der R den Rest

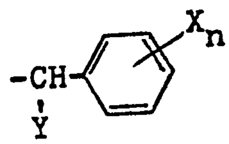

bedeutet, wobei Y für Halogen steht, erhält man nach bekannter Methode durch Umsetzung von 1 - (2 - Hydroxy - 2 - phenyl - äthyl) - 1,2,4 - triazolen der Formel II

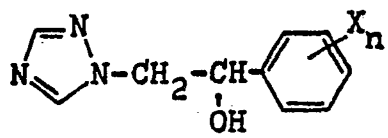

II

in der X und n die obengenannten Bedeutungen haben, mit Halogenierungsmitteln, wie Thionylchlorid, Phosphortrichlorid, Phosphortribromid, Phosphorpentachlorid, wasserfreier Flußsäure. Die dabei, beispielsweise in Form ihres Hydrochlorids, anfallenden Triazolsalze können in üblicher Weise durch Umsetzung mit einer Base, beispielsweise mit Alkalihydroxid, Alkalicarbonat oder Ammoniak, in die Basenform überführt werden.

Die Verbindungen der Formel II können durch eine für Ketone übliche Reduktionsmethode aus den entsprechenden Triazolylalkanonen der Formel III

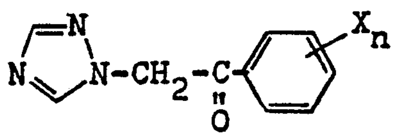

III

in der X und n die oben genannten Bedeutungen haben, hergestellt werden. Die Verbindungen der Formel III sind bekannt und können durch Umsetzung von 1,2,4-Triazol mit Verbindungen der Formel IV

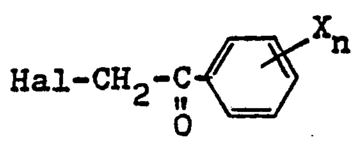

IV

in der X und n die oben genannten Bedeutungen haben und Hal für Chlor oder Brom steht, erhalten werden (DE—OS 24 31 407).

Triazolderivate der Formel I, in der R den Rest

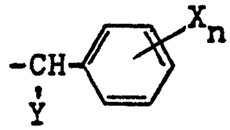

bedeutet, wobei Y für Alkylmercapto- oder Alkenylmercaptoreste steht, lassen sich durch Umsetzung von 1 - [2 - Halogen - 2 - phenyl - äthyl] - 1,2,4 - triazolen der Formel V

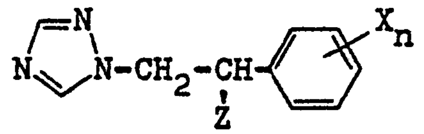

V

in der Z und X für Halogen und n für 1 oder 2 stehen, mit einem Alkyl- bzw. Alkenylmercaptid in einem Verdünnungsmittel herstellen. Das Mercaptid wird vor oder während der Umsetzung aus dem zugefügten Mercaptan durch Reaktion mit einer Base erhalten. Die entstehenden Schwefelverbindungen können mit starken Säuren in die entsprechenden Salze umgewandelt werden.

Das folgende Beispiel erläutert die Herstellung der Triazolderivate der Formel I.

Beispiel 1

Zu einer Suspension von 3,33 Gewichtsteilen Natriumhydrid in 180 Gewichtsteilen Tetrahydrofuran tropft man 12,55 Gewichtsteile Butylmercaptan. Danach tropft man eine Lösung von 27,5 Gewichtsteilen 1 - [2 - Chlor - 2 - (2,4 - dichlorphenyl) - äthyl] - 1,2,4 - triazol in 50 Teilen Tetrahydrofuran zu und rührt 3 Tage bei Raumtemperatur. Nach dem Einengen und Verrühren mit Wasser wird mit Dichlormethan extrahiert, die Dichlormethanlösung getrocknet und eingedampft, wobei 29,5 Teile rohes 1 - [2 - n - Butylmercapto - 2 - (2,4 - dichlorphenyl) - äthyl] - 1,2,4 - triazol zurückbleiben, die in Essigester gelöst werden. Beim Einleiten von Chlorwasserstoff in die Essigesterlösung fallen 25,9 Teile 1 - [2 - n - Butylmercapto - 2 - (2,4 - dichlorphenyl) - äthyl] - 1,2,4 - triazolhydrochlorid vom Schmelzpunkt 143°C aus.

Beispiel 2

Eine Mischung aus 34,2 Gewichtsteilen Benzylbromid, 13,8 Gewichtsteilen 1,2,4-Triazol und 41,4 Gewichtsteilen Kaliumcarbonat in 150 Gewichtsteilen Acetonitril wird 10 Stunden unter Rühren zum Rückfluß erhitzt. Man saugt ab, dampft das Filtrat zur Trockne ein und kristallisiert aus einem Gemisch aus Toluol und Cyclohexan um. Man erhält 21 Gewichtsteile 1 - Benzyl - 1,2,4- - triazol vom Schmelzpunkt 58°C.

Beispiel 3

129 g 1 - (2 - [2',4' - Dichlorphenyl] - 2 - hydroxy - äthyl) - 1,2,4 - triazol werden in 1 l Chloroform gelöst und unter Rühren zum Sieden erhitzt. Dazu werden langsam 90 g Thionylchlorid getropft. Nach mehrstündigem Erhitzen unter Rückfluß läßt man abkühlen und versetzt mit 1 l Toluol. Das ausgefallene Hydrochlorid wird abgesaugt und mit Petroläther gewaschen. Man erhält 150 g 1 - (2 - Chlor - 2 - [2,4 - dichlorphenyl] - äthyl) - 1,2,4 - triazol - hydrochlorid; Fp. 100°C.

Ebenso können die folgenden Verbindungen hergestellt werden:

1 - [2 - Chlor - 2 - (4-fluorphenyl) - äthyl] - 1,2,4 - triazol; viskoses Öl;
1 - [2 - Isobutylmercapto - 2 - (2,4 - dichlorphenyl) - äthyl] - 1,2,4 - triazol; viskoses Öl;
1 - [2 - Isobutylmercapto - 2 - (2,4 - dichlorphenyl) - äthyl] - 1,2,4 - triazolhydrochlorid; Fp. 114°C;
1 - [2 - Allylmercapto - 2 - (2,4 - dichlorphenyl) - äthyl] - 1,2,4 - triazol; viskoses Öl;
1 - [2 - Allylmercapto - 2 - (2,4 - dichlorphenyl) - äthyl] - 1,2,4 - triazolhydrochlorid; Fp. 103°C;
1 - (4 - Chlor - benzyl) - 1,2,4 - triazolhydrochlorid; Fp. 186 bis 187°C;
1 - (2 - Chlor - benzyl) - 1,2,4 - triazolhydrochlorid; Fp. 194 bis 195°C;
1 - (2 - Phenyl - äthyl) - 1,2,4 - triazol; Kp 101 bis 102°C/0,13 mbar;
1 - (3 - Chlor - benzyl) - 1,2,4 - triazolhydrochlorid; Fp. 161 bis 162°C;
1 - [2 - (4 - Chlorphenyl) - mercapto - 2 - (2,4 - dichlorphenyl) - äthyl] - 1,2,4 - triazol; Fp. 72°C;
1 - [2 - (4 - Chlorphenyl) - mercapto - 2 - (2,4 - dichlorphenyl) - äthyl] - 1,2,4 - triazolhydrochlorid; Fp. 176 bis 178°C;
1 - [2 - (3 - Bromphenyl) - 2 - (4 - chlorphenylmercapto) - äthyl] - 1,2,4 - triazolhydrochlorid; Fp. 172 bis 173°C;
1 - [2 - (4 - Chlorphenylmercapto - 2 - (2,4 - dichlorphenyl) - äthyl] - 1,2,4 - triazoloxalat; Fp. 155°C;
1 - [2 - Brom 2 - (2,4 - dichlorphenyl) - äthyl] - 1,2,4 - triazol; Fp. 97°C.

Beispiele für insektizide Carbamate, deren Wirksamkeit sich durch die Triazolderivate der Formel 1 steigern läßt, sind Aryl - N - methylcarbamate, N,N - Dimethylcarbamate von Hydroxyheterocyclen oder von Enolen, Oximcarbamate, beispielsweise 1 - Naphthyl - N - methyl - carbamate, m - Tolyl - N - methylcarbamat, 2 - Isopropyl - phenyl - N - methyl - carbamat, 2 - sec. - Butyl - phenyl - N - methylcarbamat, 3 - (1 - Methylbutyl) - phenyl - N - methylcarbamat, o - Chlorphenyl - N - methylcarbamat, 3,4 - Dimethyl - phenyl - N - methylcarbamat, 3,5 - Diethyl - phenyl - N - methylcarbamat, 3 - Isopropyl - 5 - methyl - phenyl - N - methylcarbamat, 6 - Chlor - 3,4 -

xylyl - N - methylcarbamat, 3,5 - Di - tert. - butyl - phenyl - N - methylcarbamat, 3,4,5 - Trimethylphenyl - N - methylcarbamat, 2 - Isopropyl - phenyl - N - methylcarbamat, 3,5 - Dimethyl - 4 - methylmercapto - phenyl - N - methylcarbamat, 4 - Dimethylamino - m - tolyl - N - methylcarbamat, 4 - Dimethylamino - 3,5 - xylyl - N - methylcarbamat, 4 - Diallylamino - 3,5 - dimethyl - phenyl - N - methylcarbamat, 2 - (1,3 - Dioxolan - 2 - yl) - phenyl - N - methylcarbamat, 4 - Benzothienyl - N - methylcarbamat, 2,3 - Dihydro - 2,2 - dimethyl - benzofuran - 7 - yl - N - methylcarbamat, 2,3 - Dimethyl - 1,3 - benzodioxol - 4 - yl - N - methylcarbamat, 1 - Isopropyl - 3 - methyl - 5 - pyrazolyl - N,N - dimethylcarbamat, 2 - Dimethylcarbamoyl - 3 - methyl - pyrazolyl - (5) - N,N - dimethylcarbamat, 2 - Dimethyl - amino - 5,6 - dimethyl - 4 - pyrimidinyl - N,N - dimethylcarbamat, 2 - (1 - Methoxy - 2 - chlor) - äthoxy - phenyl - N - methylcarbamat, 3 - [[(Dimethylamino) - carbonyl]oxy] - 1,4 - dimethyl - 4 - propyl - 2 - pyrazin - 5 - on, 2 - Methyl - 2 - (methylthio) - propionaldehyd - O - (methylcarbamoyl) - oxim, S - Methyl - N - [(methylcarbamoyl)]oxy - thioacetimidat, S - 2 - Cyanäthyl - N - [(methylcarbamoyl)oxy] - thioacetimidat, Methyl - N',N' - dimethyl - N - [(methylcarbamoyl)oxy] - 1 - thiooxamimidat, 2,4 - Dimethyl - 1,3 - dithiolan - 2 - carboxaldehyd - O - (methylcarbamoyl) - oxim, 3 - (Dimethylaminomethylen - imino) - phenyl - N - methylcarbamat, 4 - (Dimethylaminomethylen - imino) - m - tolyl - N - methylcarbamat, 2 - Äthylthiomethyl - phenyl - N - methylcarbamat.

Triazolderivate oder ihre Salze und insektizide Wirkstoffe können in den erfindungsgemäßen Mitteln in einem verhältnismäßig breiten Mengenverhältnis eingesetzt werden. In den seltensten Fällen dürfte aber ein Gewichtsverhältnis, das über den Bereich 1:10 bis 10:1 hinausgeht, sinnvoll sein. Bevorzugt ist ein Gewichtsverhältnis im Bereich von 1:5 bis 5:1.

Die erfindungsgemäßen insektiziden Mittel können gegenüber einer Vielzahl von Pflanzen- und Haushaltsschädlingen angewendet werden. Sie können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten, durch Versprühen, Vernebeln, Verstäuben, Verstreuen oder Gießen angewendet werden. Die Anwendungsformen richten sich nach den Verwendungszwecken. Triazolderivat und insektizider Wirkstoff bzw. ihre Formulierungen können dabei sowohl gemeinsam als auch getrennt ausgebracht werden.

Die Formulierungen enthalten im allgemeinen zwischen 0,1 und 95 Gew.% Synergist und Wirkstoff, vorzugsweise zwischen 0,5 und 90 Gew.%.

Die Konzentrationen von Synergist und Wirkstoff in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit einer Konzentration an Synergisten und Wirkstoff von über 95 Gew.% oder sogar eine Mischung aus Synergist und Wirkstoff allein auszubringen.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz sowie Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktiven Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der Wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Kalcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Zu den erfindungsgemäßen Mitteln können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die Verwendung der erfindungsgemäßen insektiziden Mittel erleichtert die Bekämpfung von Pflanzen- und Haushaltsschädlingen. Beispiele hierfür sind schädliche Insekten aus der Ordnung der Schmetterlinge (Lepidoptera), z.B. Plutella maculipennis (Kohlschabe), Leucoptera coffeella (Kaffeemotte), Hyponomeuta malinellus (Apfelbaumgespinstmotte), Argyresthia conjugella (Apfelmotte) Sitotroga cerealella (Getreidemotte); Phthorimaea operaculella (Kartoffelmotte), Capua reticulana (Apfelschalenwickler), Spargnothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomenella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung de Käfer (Coleoptera), z.B. Blitophaga undata (Schwarzer Rübenaaskäfer), Melanotus comminis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer) Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotars decemlineata (Kartoffelkäfer), Phaedon cochleariae (Meerrettich-Blattkäfer), Phyllotreta nemorum (Kohlerdfloh), Chaetocnema tibialis (Rübenflohkäfer), Psylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera), z.B. Mayetiola destructor (Hessenfliege), Dasyneura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege) Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritliege), Phorbia coarctata (Brachfliege), Phorbia antiqua (Zwiebelfliege), Phorbia brassicae (Kleine Kohlfliege), Pegomya hyoscyami (Rübenfliege);

aus der Ordnung der Hautflügler (Hymenoptera), z.B. Athalia rosae (Rübenblattwespe), Holocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera), z.B. Nezara viridula (Grüne Reiswanze), Eurygaster integriceps (Asiatische Getreidewanze), Blissus leucopterus (Chinch bug.) , Dysdercus cingulatus (Kapok-Wanze), Piesma quadrata (Rübenwanze), Lygus pratensis (Gemeine Wiesenwanze);

aus der Ordnung der Pflanzensauger (Homoptera), z.B. Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnenblattsauger), Trialeurodes Vaporariorum (Weiße Fliege), Aphis fabae (Schwarze Bohnenlaus), Aphis pomi (Grüne Apfellaus), Aphis sambuci (Holunderblattlaus), Aphidula nasturtii (Kreuzdornblattlaus), Cerosipha gossypii (Gurkenblattlaus), Sappaphis mali (Rosige Apfellaus), Sappaphis mala (Mehlige Birnblattlaus), Dysaphis radicola (Mehlige Apfelfalterlaus), Brachycaudus cardui (Größe Pflaumenblattlaus), Brevicoryne brassicae (Kohlblattlaus), Phorodon humuli (Hopfenblattlaus), Rhopalomyzus ascalonicus (Zwiebellaus), Myzodes Persicae (Grüne Pfirsichlaus), Myzus cerasi (Schwarze Sauerkirschenlaus), Dysaulacorthum pseudosolani (Gefleckte Kartoffellaus), Acrythosiphon onobrychis (Grüne Erbsenlaus), Macrosiphon rosae (Große Rosenblattlaus), Megoura viciae (Wickenlaus);

und die zu der Klasse der Arachnoidea gehörenden Milben und Zecken (acarina), z.B. Tetranychus urticae, Tetranychus atlanticus, Tetranychus pacificus, Paratetranychus pilosus, Bryobia praetiosa, Ixodes ricinus (Holzbock), Ornithodorus moubata, Ablyomma americanum, Dermacentor silvarum, Boophilus microplus.

Die folgenden biologischen Versuche belegen die Synergistenwirkung. Sie sind jedoch nur als Beispiele zu betrachten und umreißen in keinem Falle den ganzen erfaßten Bereich.

In den Tabellen bedeuten die Synergisten

Nr. 1

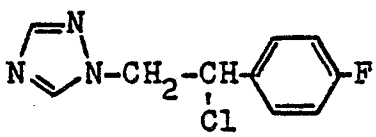

Nr. 2

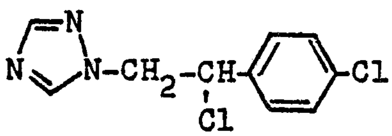

Nr. 3

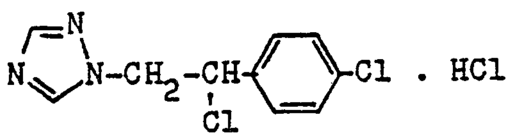

Nr. 4

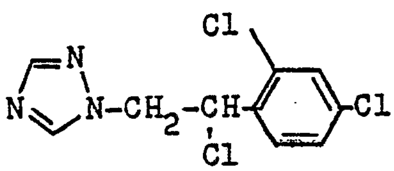

Nr. 5

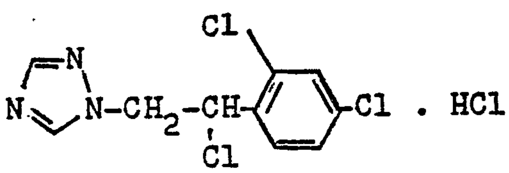

Nr. 6

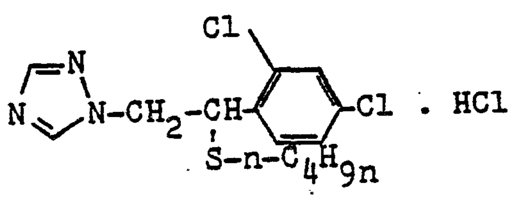

Nr. 7

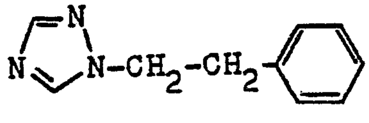

Nr. 8

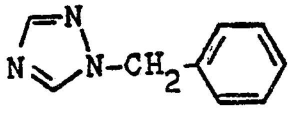

Nr. 9

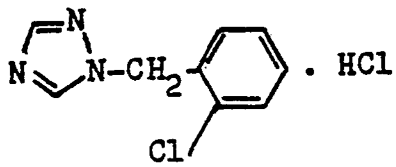

Nr. 10

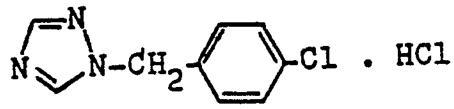

Nr. 11

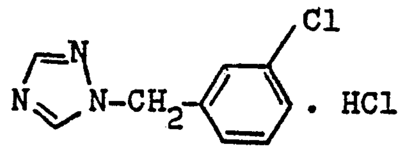

Nr. 12

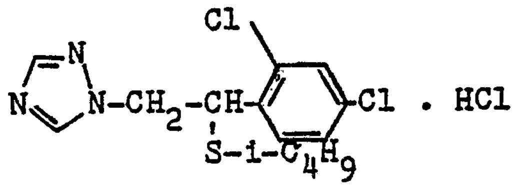

Nr. 13

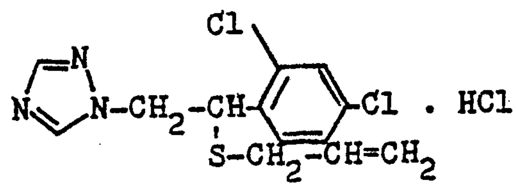

Nr. 14

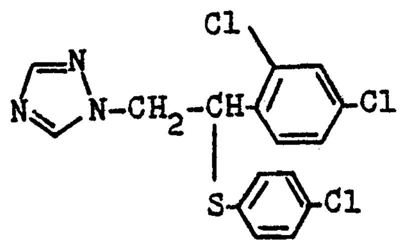

Folgende Insektizide werden verwendet:
1-Naphthyl-N-methylcarbamat (Carbaryl),
2-Dimethylamino-5,6-di-methyl-4-pyrimidinyl-N,N-dimethylcarbamat (Pirimicarb),
3,5-Diäthyl-phenyl-N-methylcarbamat (Fenethcarb),
2-Isopropoxy-phenyl-N-methylcarbamat (Propoxur).

Beispiel A

Synergistenwirkung bei Schaben (Blatta orientalis)

Der Boden eines 1 l-Glases wird mit der acetonischen Lösung des Insektizides und des Synergisten in den vorgesehenen Mengenverhältnissen behandelt. Nach Verdunsten des Lösungsmittels belegt man die Gläser mit je 5 ausgewachsenen Schaben (Blatta orientalis) und bestimmt nach 48 Stunden die Mortalitätsrate. Die Aufwandmengen liegen in logarithmischer Stufung und erlauben die Erstellung einer Dosis-Effekt-Kurve. Die LD 50 wird graphisch ermittelt und dient als Grundlage der Bewertung.

I. Als Insektizid wird Carbaryl eingesetzt; das Verhältnis insektizid: Synergist beträgt 1:5 Gewichtsteile.

| Wirkstoff | LD 50 | | |
|---|---|---|---|
| Carbaryl | 0,25 | mg | |
| Nr.1 | > 10,0 | mg | |
| Carbaryl + Nr. 1 | 0,02 | mg + 0,1 | mg |
| Nr. 2 | 3,0 | mg | |
| Carbaryl + Nr. 2 | 0,025 | mg + 0,13 | mg |
| Nr. 3 | 3,0 | mg | |
| Carbaryl + Nr. 3 | 0,021 | mg + 0,105 | mg |
| Nr. 4 | > 5,0 | mg | |
| Carbaryl + Nr. 4 | 0,03 | mg + 0,15 | mg |
| Nr. 5 | > 5,0 | mg | |
| Carbaryl + Nr. 5 | 0,034 | mg + 0,17 | mg |
| Nr. 6 | > 5,0 | mg | |
| Carbaryl + Nr. 6 | 0,1 | mg + 0,5 | mg |
| Nr. 7 | > 2,0 | mg | |
| Carbaryl + Nr. 7 | 0,013 | mg + 0,065 | mg |
| Nr. 8 | > 2,0 | mg | |
| Carbaryl + Nr. 8 | 0,027 | mg + 0,135 | mg |
| Nr. 9 | > 2,0 | mg | |
| Carbaryl + Nr. 9 | 0,022 | mg + 0,11 | mg |
| Nr. 10 | > 2,0 | mg | |
| Carbaryl + Nr. 10 | 0,015 | mg + 0,075 | mg |
| Nr. 11 | > 2,0 | mg | |
| Carbaryl + Nr. 11 | 0,014 | mg + 0,07 | mg |
| Nr. 12 | > 2,0 | mg | |
| Carbaryl + Nr. 12 | 0,035 | mg + 0,175 | mg |
| Nr. 13 | > 2,0 | mg | |
| Carbaryl + Nr. 13 | 0,03 | mg + 0,15 | mg |

II. Einsatz unterschiedlicher Insektizide bei verschiedenen Mischungsverhältnissen

| Wirkstoff | Gewichtsverhältnis Wirkstoff : Synergist | LD 50 |
|---|---|---|
| Carbaryl | | 0,25 mg |
| Nr. 1 | | ca. 10 mg |
| Carbaryl + Nr. 1 | 5:1 | 0,009 mg + 0,018 mg |
| Carbaryl + Nr. 1 | 1:1 | 0,07 mg + 0,07 mg |
| Pirimicarb | | ca. 25 mg |
| Pirimicarb + Nr. 1 | 5:1 | 7,0 mg + 1,5 mg |
| Pirimicarb + Nr. 1 | 1:1 | 0,9 mg + 0,9 mg |
| Pirimicarb + Nr. 1 | 1:5 | 0,52 mg + 2,6 mg |
| Fenethcarb | | 0,66 mg |
| Fenethcarb + Nr. 1 | 5:1 | 0,13 mg + 0,026 mg |
| Fenethcarb + Nr. 1 | 1:1 | 0,086 mg + 0,086 mg |
| Fenethcarb + Nr. 1 | 1:5 | 0,038 mg + 0,19 mg |

Beispiel B

Synergistenwirkung bei Stubenfliegen (Musca domestica)

Beide Hälften einer Petrischale von 10 cm Durchmesser werden mit insgesamt 2 ml der acetonischen Wirkstofflösungen ausgekleidet. Nach Verdunsten des Lösungsmittels (ca. 30 Minuten) bringt man je 10 Fliegen in die Schalen.

Die Motalitätsrate wird nach 4 Stunden und die LD 50 graphisch ermittelt.

| Wirkstoff | Gewichtsverhältnis Wirkstoff : Synergist | LD 50 |
|---|---|---|
| Carbaryl | | 7,0 mg |
| Nr. 1 | | 5,0 mg |
| Carbaryl + Nr. 1 | 5:1 | 0,8 mg + 0,16 mg |
| Carbaryl + Nr. 1 | 1:5 | 0,2 mg + 1.0 mg |
| Nr. 8 | | > 2,0 mg |
| Carbaryl + Nr. 8 | 1:1 | 0,1 mg + 0,1 mg |

Beispiel C

Synergistenwirkung bei Stubenfliegen (Musca domestica)

Verwendet werden adulte Stubenfliegen (4 Tage nach dem Schlüpfen). Diese Tiere erhalten in leichter $CO_2$-Narkose je 1 $\mu$l der acetonischen Wirkstofflösung auf das ventrale Abdomen appliziert.

20 Fliegen mit gleicher Behandlung bringt man in einen Cellophanbeutel (Volumen ca. 500 ml). Nach 4 Stunden ermittelt man die Mortalitätsrate, erstellt aus den unterschiedlichen Konzentrationen die Dosis-Mortalitäts-Kurve und ermittelt die LD 50.

| Wirkstoff | LD 50 |
|---|---|
| Nr. 1 | 10,0 µg/Fliege unwirksam |
| Fenethcarb | 0,16 µg/Fliege |
| Fenethcarb + Nr. 1 | 0,09 µg + 0,45 µg/Fliege |

Beispiel D

Synergistenwirkung bei Raupen der Kohlschabe (Plutella maculipennis)

Blätter von jungen Kohlpflanzen werden 3 Sekunden in die wäßrige Wirkstoffemulsion getaucht und nach kurzem Abtropfen auf einen angefeuchteten Filter in eine Petrieschale gelegt. Das Blatt wird darauf mit 10 Raupen des 4. Stadiums belegt. Nach 48 Stunde wird die Mortalitätsrate in den einzelnen Konzentrationen festgestellt und die LC 50 ermittelt.

| Wirkstoff | LC 50 |
|---|---|
| Nr.1 | 0,25 % |
| Carbaryl | 0,16 % |
| Carbaryl + Nr. 1 | 0,03 % + 0,03 % |
| Pirimicarb | 0,25 % |
| Pirimicarb + Nr. 1 | 0,11 % + 0,11 % |
| Propoxur | 0,025 % |
| Propoxur + Nr. 1 | 0,006 % + 0,006 % |

Beispiel E

Synergistenwirkung bei Kornkäfer (Sitophilus granaria)

Petrischalen von 10 cm Durchmesser werden mit acetonischer Wirkstofflösung ausgekleidet. Nach Verdunsten des Lösungsmittels belegt man die Schalen mit 50 Kornkäfern.

Nach 4 Stunden werden die Käfer in unbehandelte Gefäße überführt. Die Mortalitätsrate wird nach 24 Stunden ermittelt. Dabei wird festgestellt, wieviele Käfer in der Lage sind, nach diesem Zeitpunkt innerhalb von 60 Minuten ein unbehandeltes Pappschälchen (Durchmesser: 40 mm, Höhe: 10 mm) zu verlassen.

| Wirkstoff | LD 50 |
|---|---|
| Carbaryl | 2,5 mg |
| Nr. 1 | 2,0 mg unwirksam |
| Carbaryl + Nr. 1 | 0,1 mg + 0,1 mg |
| | 0,055 mg + 0,275 mg |
| Nr. 8 | 2 mg unwirksam |
| Carbaryl + Nr. 8 | 0,4 mg + 2 mg |
| Nr. 10 | 5 mg unwirksam |
| Carbaryl + Nr. 10 | 1,3 mg + 6,5 mg |
| Propoxur | 7 mg |
| Propoxur + Nr. 1 | 1,25 mg + 1,25 mg |
| Nr. 12 | 5 mg unwirksam |
| Carbaryl + Nr. 12 | 0,22 mg + 1,1 mg |
| Nr. 13 | 5 mg unwirksam |
| Carbaryl + Nr. 13 | 0,11 mg + 0,55 mg |

**Patentansprüche**

1. Insektizides Mittel, gekennzeichnet durch einen Gehalt an mindestens einem Triazolderivat der Formel I

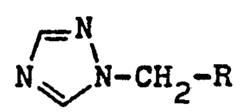

I

in der
R gegebenenfalls durch Halogen substituiertes Phenyl oder den Rest

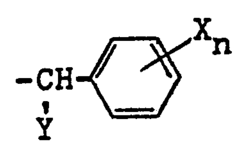

bedeutet,
wobei
Y für Wasserstoff, Halogen, Alkylmercapto mit 1 bis 6 Kohlenstoffatomen oder Alkenylmercapto mit 2 bis 6 Kohlenstoffatomen oder einen gegebenenfalls durch Halogen substituierten Phenylmercaptorest,
X für Halogen und
n für 0, 1 oder 2 stehen, mit der Maßgabe, daß n nicht 0 bedeutet, wenn Y für Halogen steht, oder einem Salz eines triazolderivats der Formel 1 und mindestens einem insektiziden Wirkstoff aus der Gruppe der Carbamate.

2. Insektizides Mittel nach Anspruch 1, dadurch gekennzeichnet, daß es ein Triazolderivat der Formel 1 enthält, in der Y für Chlor steht.

3. Insektizides Mittel nach Anspruch 1, dadurch gekennzeichnet, daß es ein Triazolderivat der Formel I enthält, in der Y für Alkylmercapto mit 1 bis 6 Kohlenstoffatomen steht.

4. Insektizides Mittel nach Anspruch 1, dadurch gekennzeichnet, daß es als Triazolderivat der Formel I 1 - [2 - Chlor - 2 - (4 - fluorphenyl) - äthyl] - 1,2,4 - triazol enthält.

5. Insektizides Mittel nach Anspruch 1, dadurch gekennzeichnet, daß das Gewichtsverhältnis des Triazolderivats der Formel I oder seines Salzes und des Carbamats zueinander 1:10 bis 10:1 beträgt.

**Revendications**

1. Insecticide, caractérisé par le fait qu'il contient au moins un dérivé de triazole de formule I

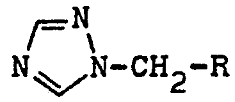

I

dans laquelle
R représente un groupe phényle éventuellement substitué par un halogène ou le reste

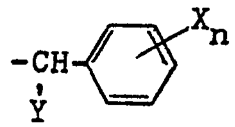

Y représentant l'hydrogène, un halogène, un reste alkylmercapto en $C_1$ à $C_6$, ou alcénylmercapto en $C_2$ à $C_6$, ou un reste phénylmercapto éventuellement substitué par un halogène
X représent un halogène et
*n* étant 0, 1 ou 2, sous réserve que *n* ne soit pas 0 si Y est un halogène,
ou un sel de dérivé de triazole de formule I et au moins un principe actif insecticide du groupe des carbamates.

2. Insecticide selon la revendication 1, dans lequel Y de la formule I est du chlore.

3. Insecticide selon la revendication 1, dans lequel Y de la formule I représente un reste alkylmercapto en $C_1$ à $C_6$.

4. Insecticide selon la revendication 1, dans lequel de dérivé de triazole de formule I est le 1-[2 - chloro - 2 - (4 - fluorophényl) - éthyl] - 1,2,4 - triazole.

5. Insecticide selon la revendication 1, dans lequel le rapport en poids entre le dérivé de triazole de formule I ou son sel et le carbamate est compris entre 1/10 et 10/1.

**Claims**

1. An insecticidal agent characterized by a content of at least one triazole derivative of the formula I

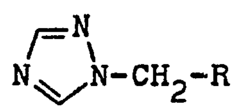

I,

where R denotes unsubstituted or halogen-substituted phenyl, or

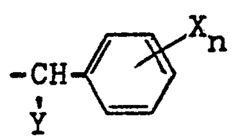

Y denoting hydrogen, halogen, alkylmercapto of 1 to 6 carbon atoms, alkenylmercapto of 2 to 6 carbon atoms, or unsubstituted or halogen-substituted phenylmercapto, X denoting halogen, and n denoting 0, 1 or 2, with the proviso that n does not denote 0 when Y is halogen, or a salt of a triazole derivative of the formula I, and of at least one insecticidal active ingredient selected from the group consisting of the carbamates.

2. An insecticidal agent as claimed in claim 1, characterized in that it contains a triazole derivative of the formula I in which Y denotes chlorine.

3. An insecticidal agent as claimed in claim 1, characterized in that it contains a triazole derivative of the formula I in which Y denotes alkylmercapto of 1 to 6 carbon atoms.

4. An insecticidal agent as claimed in claim 1, characterized in that it contains 1 - [2 - chloro - 2 - (4 - fluorophenyl) - ethyl] - 1,2,4 - triazole as the triazole derivative of the formula I.

5. An insecticidal agent as claimed in claim 1, characterized in that the weight ratio of the triazole derivative of the formula I, or a salt thereof, to the carbamate is from 1:10 to 10:1.